# EUROPÄISCHE PATENTSCHRIFT

(11) **EP 3 506 954 B1**
(45) Veröffentlichungstag und Bekanntmachung des Hinweises auf die Patenterteilung: **29.09.2021**
(21) Anmeldenummer: 17755187.6
(22) Anmeldetag: 22.08.2017
(51) Int. Cl.: A61L 27/42, A61L 27/48, A61L 27/58

(54) **FASERVERSTÄRKTES BIORESORBIERBARES IMPLANTAT UND VERFAHREN ZU DESSEN HERSTELLUNG**
FIBER-REINFORCED BIORESORBABLE IMPLANT AND METHOD FOR PRODUCING SAME
IMPLANT BIORÉSORBABLE RENFORCÉ PAR DES FIBRES ET PROCÉDÉ POUR SA FABRICATION

(30) Priorität: 01.09.2016 DE 102016116387
(43) Veröffentlichungstag der Anmeldung: 10.07.2019
(73) Patentinhaber: Karl Leibinger Medizintechnik GmbH & Co. KG, 78570 Mühlheim (DE)
(72) Erfinder: AKSU, Adem, 78570 Mühlheim (DE); REINAUER, Frank, 78570 Mühlheim (DE); WOLFRAM, Tobias, 78570 Mühlheim (DE); GABELE, Lorenz, 78570 Mühlheim (DE)
(74) Vertreter: Winter, Brandl - Partnerschaft mbB
(86) Internationale Anmeldenummer: PCT/EP2017/071129
(87) Internationale Veröffentlichungsnummer: WO 2018/041668

(56) Entgegenhaltungen:
- WO-A1-2012/084214
- US-A1- 2003 049 329
- US-A1- 2011 280 924

## Beschreibung

Die Erfindung betrifft ein bioresorbierbares Implantat zum Ergänzen oder Ersetzen von Hartgewebe und / oder Weichgewebe, wie z.B. Knorpel, Knochen oder anderem Gewebe. Das bioresorbierbare Material weist zumindest eine verstärkende Faser auf. Diese zumindest eine verstärkende Faser ist vorzugsweise als ein verstärkendes Faserbündel / ein verstärkendes Fasergerüst oder ein verstärkendes Faserkonstrukt ausgebildet. Die zumindest eine verstärkende Faser ist aus einer ersten Materialkomponente aufgebaut und ist nach einer Vermischung mit einer zweiten Materialkomponente in einer Matrix eingebettet. Die zweite Materialkomponente liegt zum Zeitpunkt der Vermischung mit der zumindest einen verstärkenden Faser granulatartig oder pulverförmig vor.

Das Material der ersten Materialkomponente enthält zumindest eines oder eine Kombination der Elemente der Gruppe aus Seide, Chitosan, Collagen, Polycaprolacton, Poly-DL-Lactide, Polylactid-co-Glycolid, Polyclycolide, Polyurethane und Polypropylen, wobei die Gesamtheit an Fasern zumindest zwei der Elemente der Gruppe aus Seide, Chitosan, Collagen, Polycaprolacton, Poly-DL-Lactide, Polylactid-co-Glycolid, Polyglycolide, Polyurethane und Polypropylen enthält.

Es sei also noch einmal betont, dass unter Fasern hier auch Faserbündel und Fasergerüste sowie Faserkonstrukte verstanden werden.

Ebenso betrifft die Erfindung ein Verfahren zur Herstellung des bioresorbierbaren Implantats.

Aus dem Stand der Technik sind einerseits bioresorbierbare Implantate bekannt, die aus nur einem Rohmaterial hergestellt sind. Um eine Verbesserung der biologischen Reaktionen auf die Degradation der resorbierbaren Materialien zu bewirken, haben sich andererseits auf dem Gebiet der Implantate Technologien durchgesetzt, die auf mehreren Materialien aufgebaut sind. Den verbesserten biologischen Reaktionen steht hierbei jedoch ein Verlust an mechanischer Festigkeit gegenüber, was unter Umständen zu einem frühzeitigen Versagen des Implantates unter mechanischer Belastung führt. So weisen solche Implantate, die für die Regeneration von Defekten eingesetzt werden, in der Regel keine ausreichende mechanische Stabilität auf, um das zu ersetzende Gewebe unmittelbar nach dem operativen Einsetzen vollumfänglich zu ersetzen.

Ein gattungsgemäßes Implantat ist aus der deutschen Patentanmeldung 10 2010 034 471 A1 bekannt. Diese offenbart ein Implantat, das einen Faden umfasst, der einen länglichen, vorzugsweise geflochtenen, Fadenkörper und eine den Fadenkörper zumindest teilweise umgebende Beschichtung aufweist. Der Faden jener Patentanmeldung ist aus Polyethylen und/oder Polypropylen aufgebaut, während die Beschichtung aus einem resorbierbaren Material und gegebenenfalls aus Additiven besteht.

Auch aus der europäischen Patentschrift 1 537 883 B1 ist ein Implantat bekannt. Dieses ist auf die Reparatur von Gewebeschäden und -defekten gerichtet und weist ein biokompatibles Gerüst auf, das ein verstärkendes Material umfasst.

In der europäischen Patentanmeldung 2 081 020 A2 ist ein Gewebeimplantat offenbart, das aus bioresorbierbaren Komponenten und/oder aus nicht-bioresorbierbaren Komponenten aufgebaut ist. Als nicht-bioresorbierbare Komponente kommt beispielsweise natürliche oder synthetische Seide in Frage.

Auch die US-Patentanmeldung mit der Nummer 2004/0054372 A1 ist auf biologisch abbaubare Verbundwerkstoffe als Implantat gerichtet. Jenes Implantat umfasst ein biodegradierbares, faserverstärktes Komposit, eine Matrix sowie Fasern.

WO2012084214 beschreibt Knochenersatzmaterial, enthaltend poröses biphasisches Calciumphosphat/Hydroxylapatit und Collagen.

US2011280924 beschreibt bioabbaubares Knochenfüllmaterial, enthaltend chemisch quervernetzte Collagenfasern und darin verteilte unterstützende Teilchen, umfassend Biokeramik und/oder Bioglas.

Nachteilig an diesem Stand der Technik ist, dass durch die Anpassung der Implantatgeometrien an individuelle Eigenschaften die eingesetzte Materialmenge ansteigt. Neben der abnehmenden Wirtschaftlichkeit aufgrund des erhöhten Materialeinsatzes bewirkt dies eine Verschlechterung der biologischen Prozesse, was klinische Resultate negativ beeinträchtigt. Denn bekanntermaßen können konzentrationsbedingte negative klinische Reaktionen durch eine vergrößerte Menge an abzubauenden Substanzen aus dem Implantat hervorgerufen werden.

Ebenso sind die zu verbindenden Verbundwerkstoffe im Stand der Technik hauptsächlich mittels einer sogenannten Prepreg-Technologie (Kurzform für "preimpregnated fibres") hergestellt. Bei diesen handelt es sich um mit Reaktionsharzen vorimprägnierte textile Faser-Matrix-Halbzeuge, die zur Herstellung von Implantaten unter Temperatur und Druck ausgehärtet werden. Diese ist kostenintensiv und zeitaufwendig. US2003049329 beschreibt bioabbaubares Implantat- Keramikmaterial, hergestellt aus einem Calciumphosphatpulver und enthaltend eine Calciumphophatmatrix, die durch in die Matrix eingebettete Polylactid-Fasern verstärkt ist.

Die Aufgabe der Erfindung besteht somit darin, die aus dem Stand der Technik bekannten Nachteile zu beheben oder zumindest zu mildern und insbesondere ein Implantat zur Verfügung zu stellen, das bei möglichst wenig Materialeinsatz hohe Festigkeitswerte, rasche bioresorbierbare Eigenschaften sowie eine wirtschaftliche Herstellung ermöglicht.

Dies wird dadurch gewährleistet, dass die zweite Materialkomponente zum Zeitpunkt der Vermischung mit der zumindest einen verstärkenden Faser / dem Faserbündel / dem Fasergerüst oder Faserkonstrukt granulatartig oder pulverförmig vorliegt. Somit liegt die erste Materialkomponente in Faserform / Faserbündelform oder Fasergerüst- oder Faserkonstruktform und die zweite beispielsweise in Pulver- oder Granulatform vor, welche nun in unterschiedlichen Mengen und Stoffzusammensetzungen miteinander zu vermischen sind. So ist eine hohe Flexibilität der zu erreichenden Eigenschaften gewährleistet, da die Vermischung von zumindest einer Faser / einem Faserbündel / einem Fasergerüst oder Faserkonstrukt und von einem Pulver oder Granulat bei einer hohen Zuverlässigkeit individuell ausgestaltbar ist.

Die Erfindung wird durch die unabhängigen Ansprüche definiert.

Vorteilhafte Ausführungsformen sind Gegenstand der Unteransprüche und werden nachfolgend näher erläutert.

So ist es von Vorteil, wenn die Matrix aus der zweiten Materialkomponente und zumindest einer weiteren Materialkomponente aufgebaut ist. Dies bewirkt, dass die Eigenschaften der Matrix, etwa bezüglich der Festigkeit oder bezüglich der Abbaukinetik oder der biologischen Adaption, mittels Zugabe einer weiteren Komponente gezielt beeinflussbar sind.

Sobald die zweite Materialkomponente keramische phosphatbasierte Komponenten umfasst, ist eine hohe Festigkeit der Matrix, die eine Gerüstfunktion einnimmt, sichergestellt. Dies erhöht die Zuverlässigkeit des Implantats und dessen biologische Verträglichkeit. Erfindungsgemäß enthält die Gesamtheit an Fasern zumindest zwei der Elemente der Gruppe aus Seide, Chitosan, Collagen, Polycaprolacton, Poly-DL-Lactide, Polylactid-co-Glycolid, Polyclycolide, Polyurethane und Polypropylen. Hierbei ist es sowohl möglich, dass eine Faser aus mehreren Werkstoffen aufgebaut ist, als auch dass einzelne Fasern den gleichen, jedoch untereinander unterschiedliche Werkstoffe aufweisen. Jedes einzelne der Elemente aus der vorstehenden Gruppe weist hierbei seine individuellen Vorteile auf, die aus der Materialwissenschaft bekannt sind. So ist die Wahl, welches beziehungsweise welche der Elemente zu wählen ist/sind, von den jeweiligen Rahmenbedingungen abhängig. Als Einflussfaktoren seien an dieser Stelle die Festigkeit, die biologische Verträglichkeit, die Wirtschaftlichkeit sowie das Volumen- und das Masseverhältnis der Faserkomponente zur Matrix angeführt.

Ein weiterer Vorteil entfaltet sich, wenn die Faser zumindest eine Erhöhung und/oder Vertiefung aufweist, um eine Interaktionsoberfläche zwischen der Matrix und der Faser zu erhöhen. Dies ermöglicht einen robusten Sitz der Faser in der Matrix. So hält das Implantat auch bei unerwartet hohen äußeren und inneren Krafteinwirkungen der Belastung stand.

In einer vorteilhaften Ausführungsform sind in der Matrix mehrere Festkörperpartikel angeordnet, die eine Steuerung einer Resorptionszeit ermöglichen, wobei die Festkörperpartikel einen Masseanteil von 5% bis 25% gemessen an der Masse des bioresorbierbaren Materials / Implantats aufweisen. Je höher der Masseanteil der Festkörperpartikel in der Matrix, desto höher ist der zeitliche Einfluss, den sie ausüben. Hierbei ist abzuwägen, wieviel Masseprozent unter Einhaltung der Festigkeitsrahmenbedingungen und unter Beachtung der biologischen Eigenschaften einsetzbar sind. Dadurch, dass die zweite Materialkomponente zum Zeitpunkt der Vermischung pulver- und/oder granulatartig ausgeformt ist, ist die Zumischung der Festkörperpartikel in der Herstellung des Implantats ohne hohen Zusatzaufwand realisierbar.

Auch wenn eine Vermengung der einzelnen Materialkomponenten über eine generative, subtraktive und 3D-formgebende Fertigung ermöglicht ist, ist eine präzise und zuverlässige Herstellung des bioresorbierbaren Materials begünstigt. Das generative Schichtbauverfahren wird durch die Pulver- / Granulatform sowie die Fadenform begünstigt. Jene generative Fertigung bewirkt, dass Stützstrukturen, wie sie bei anderen Verfahren des Rapid-Prototyping nötig sind, entfallen, was sich unter anderem positiv auf die einzusetzende Materialmenge auswirkt.

Dies gilt analog auch für weitere 3-D-formgebende Verfahren, wie z.B. Compression Molding. Als weitere Möglichkeit zur Vermengungen der einzelnen Bestandteile bietet sich LCM ("lithographic based Ceramic Manufacturing") oder Electrospinning an. Weiterhin entstehen Vorteile, wenn die zum Zeitpunkt der Vermischung granulatartigen oder pulverförmigen Partikel der zweiten Materialkomponente nach einem vorbestimmten Zeitfenster eine ganzheitliche und ineinander übergehende Form annehmen. So entfällt jegliche Pulverform der zweiten Materialkomponente bei einer entsprechenden Nachbearbeitung, was sich begünstigend auf einen Kraftverschleiß innerhalb des Implantats auswirkt, da keine materialinternen Phasengrenzen mehr vorhanden sind.

In einer weiteren bevorzugten Ausführungsform enthalten die zum Zeitpunkt der Vermischung granulatartigen oder pulverförmigen Partikel der zweiten Materialkomponente zumindest eines der Elemente der Gruppe aus Magnesium, Calcium, Hydroxylapatit, alpha- und/oder beta-Tricalciumphosphat und Kalk, um das Abbauverhaltung des bioresorbierbaren Implantats gezielt zu beeinflussen. Auf diese Wiese ist die Bioresorption des Implantats abhängig von einem Gesundheitszustand eines Patienten variabel anpassbar, um eine komplikationslose und rasche Heilung zu gewährleisten.

Ebenfalls Teil der Erfindung ist ein Verfahren zum Herstellen des oben beschriebenen bioresorbierbaren Implantats. Dieses weist verschiedene Schritte auf, die vorzugsweise zeitlich nacheinander abgearbeitet werden. Nach einem Bereitstellen der Faser aus der ersten Materialkomponente folgt ein Bereitstellen von granulatartigen oder pulverförmigen Partikeln der zweiten Materialkomponente. Sind beide Materialkomponenten hinsichtlich der äußeren Bedingungen, wie ihrer Anordnung oder ihrer Temperatur, in einen Zustand gebracht, in dem sie für eine Vermischung bereit sind, findet diese statt, um aus der ersten Materialkomponente und der zweiten Materialkomponente das bioresorbierbare Implantat zu erhalten. Dieses weist eine dreidimensionale Geometrie auf und wird nachbearbeitet, um in seiner Form und/oder Oberflächenbeschaffenheit, komplementär zu dem zu ersetzenden Hartgewebe ausgestaltet zu sein.

Das erfindungsgemäße Verfahren ist wahlweise durch einen Schritt eines Zumischens einer weiteren Komponente erweitert, um das Implantat hinsichtlich seiner Abbaukinetik, sowie seiner biologischen Interaktion mit dem Körper des Patienten zu optimieren.

Ebenso Teil der Erfindung ist, dass die zweite Materialkomponente die Faser der ersten Materialkomponente derart umgibt, dass eine dreidimensionale Ausdehnung und Geometrie des Implantats definiert ist. Diese Ausdehnung / Geometrie ist variabel ausgestaltbar und somit optimal an die sich ändernden Bedingungen anpassbar. Erfindungsgemäß liegt der Faseranteil in Masseprozent des Implantats zwischen 5% und bis zu 95%. Besonders bevorzugt sind Ausführungen, in denen der Masseanteil bei 5%, 15%, 20%, 30 bis 55% oder 60 bis 95% liegt.

Da die Dichte der unterschiedlichen Materialkomponenten nicht konstant ist, entspricht der Faseranteil in Volumenprozent nicht zwangsläufig dem in Masseprozent. In Volumenprozent beträgt der Faseranteil zwischen 5% und bis zu 95%. Besonders bevorzugt sind Ausführungen, in denen der Volumenanteil bei 5%, 15%, 20%, 30 bis 55% oder 60 bis 95% liegt.

Die Fasern sind dabei vorzugsweise so angeordnet, dass sie die Festigkeitseigenschaften des Implantats optimieren. Zusätzlich ist es möglich, das bioresorbierbare, faserverstärkte Implantat mit weiteren Materialien zu versehen. Diese sind vorteilhaft in partikulärer Form enthalten. Beispiele für jene Partikel seien mit Magnesium, Eisen, Barium, Strontium, Calcium, Hydroxylapatit, alpha- und/oder beta-Tricalciumphosphat und Kalk gegeben. Es ist hierbei möglich, dass alle Partikel aus dem Gleichen Element / Material aufgebaut sind oder dass die einzelnen Partikel unterschiedlich sind. Der jeweilige Anwendungsfall, also die Situation des Patienten, entscheidet darüber, ob und welche Partikel eingesetzt werden. Jeder dieser Zweitstoffe ist hierbei derart eingesetzt, dass er auf den natürlichen Knochenaufbau unterstützend wirkt.

Vorteilhaft beträgt der Anteil jener Partikel an der Gesamtmasse des Implantats zwischen 5% und 25%, etwa 10%, 15% oder 20%.

Hinsichtlich seines Volumens sei der Anteil jener Partikel an der Gesamtmasse vorteilhaft mit 5% bis 25%, etwa 10%, 15% oder 20% bemessen.

Die Festigkeit des Implantats, das Partikeln aufweist, hängt damit zusammen, welche Geometrien die Partikel aufweisen. Auch die Abbaukinetik wird hiervor beeinflusst. Vorzugsweise sind die Partikel im Wesentlichen kugelförmig ausgestaltet. Hierbei sind Kugeldurchmesser von 30µm bis 60µm üblich. Ebenso sind deutlich kleinere Kugeldurchmesser von 30nm bis 60nm erfindungsgemäß einsetzbar.

In einer vorteilhaften Ausführungsform beträgt der Partikeldurchmesser 1µm bis 10µm, weiter bevorzugt 15µm bis 25µm und weiter bevorzugt 50µm bis 150µm. Der jeweilige Anwendungsfall, also die Situation des Patienten, entscheidet darüber, welche Partikelgröße eingesetzt wird.

Hinsichtlich der zumindest einen Faser ist ebenso die Geometrie variierbar. Bevorzugt weisen die Fasern eine Länge von 1mm bis 10mm auf. In größeren Implantaten beträgt die Faserlänge 50mm bis 100mm.

Die Faser weist in einer Ausführungsform einen kreisrunden Querschnitt auf. Dieser hat einen Durchmesser von rund 30µm. Ebenso ist ein Faserdurchmesser von 10nm bis 1µm möglich. In einem weiteren Ausführungsbeispiel beträgt die Dimension des Faserdurchmessers 5µm bis 15µm und in noch einem weiteren 100µm bis 500µm.

Die Gesamtheit der Fasern setzt sich bevorzugt zu einem Faserkonstrukt zusammen, das z.B. netzartig zueinander orientiert ist. Hierbei ist diese Struktur in einer Ebene oder auch dreidimensional ausgestaltbar. Das Faserkonstrukt besitzt eine Ausrichtungsorientierung von einzelnen Teilfasern, welche miteinander netzartig verwoben sind.

Bevorzugt weisen die Fasern eine strukturelle Oberflächentopographie auf, um die Interaktionsfläche zwischen den einzelnen Fasern und der Matrix zu intensivieren / zu vergrößern. Dies erhöht die mechanische Stabilität / Robustheit / Festigkeit des erfindungsgemäßen Implantats.

Das vorstehend als Fasermaterial vorgestellte Chitosan zeichnet sich dadurch aus, dass es neben der verstärkenden Funktion auch eine antibakterielle Wirkung entfaltet.

Durch die Vermengung der Partikel, wie Magnesium, Magnesium-Calcium-Zink (MgCaZn), Eisen, Barium, Strontium, Calcium, Hydroxylapatit, alpha- und/oder beta-Tricalciumphosphat und Kalk, mit den entsprechenden Fasern ist eine Zeitzone / eine zeitliche Entwicklung derart regulierbar, dass das Abbauverhalten des resorbierbaren Materials beschleunigt werden kann oder, je nach Bedarf, auch verlangsamt werden kann.

Erfindungsgemäß ist es ebenso möglich, Oberflächenmodifikationen vorzunehmen, um die antibakterielle Wirkung des Implantats zu verbessern und das Einwachsverhalten zu optimieren. Hierbei sind bevorzugt die Bestandteile Magnesium, Polyethylen, Polypropylen, Polyetheretherketon, Hydroxylapatit, alpha- und/oder beta-Tricalciumphosphat und Kalk zu modifizieren, um im Zusammenspiel mit den Fasern das gewünschte Verhalten des Implantats hervorzurufen.

Die Erfindung wird nun nachfolgend anhand von Figuren näher erläutert, in welchem Zusammenhang auch verschiedene Ausführungsbeispiele erläutert sind. Diese zeigen:
- Fig. 1:: einen Schnitt durch ein erfindungsgemäßes Implantat in einem Zustand kurz nach einer Vermischung; und
- Fig. 2:: einen Schnitt gemäß Fig. 1 in einem späteren Zustand.

Die Figuren sind lediglich schematischer Natur und dienen ausschließlich dem Verständnis der Erfindung.

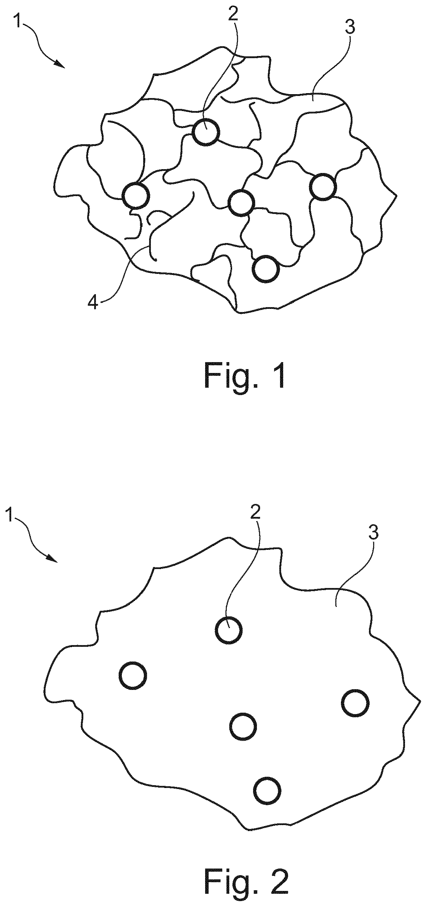

Fig. 1 zeigt ein bioresorbierbares Implantat 1 zum Ergänzen oder Ersetzen von Hartgewebe mit zumindest einer verstärkenden Faser 2. Diese Faser 2 wiederum weist erfindungsgemäß zumindest eines der Elemente der Gruppe aus Seide, Chitosan, Collagen, Polycaprolacton, Poly-DL-Lactide, Polylactid-co-Glycolid, Polyclycolide, Polyurethane und Polypropylen auf, wobei die Gesamtheit an Fasern zumindest zwei der Elemente der Gruppe aus Seide, Chitosan, Collagen, Polycaprolacton, Poly-DL-Lactide, Polylactid-co-Glycolid, Polyglycolide, Polyurethane und Polypropylen enthält. Die Faser 2 ist in einem Matrixmaterial 3 eingebettet, um mit diesem ein solches Implantat 1 zu formen, das sowohl entlang der Längs- als auch entlang der Querrichtung hohe Festigkeitswerte aufweist. Das Matrixmaterial 3 ist zum Zeitpunkt der Vermischung mit der Faser 2 granulatartig oder pulverförmiug .

Dies ist an den Phasengrenzen 4 in Fig. 1 erkennbar.

In Fig. 2 ist ein Zustand dargestellt, in dem die ursprünglich granulatartige Zusammensetzung des Matrixmaterials 3 vollends aufgehoben ist, sodass das Implantat 1 lediglich eine homogene Matrix 3 aufweist, in der verstärkende Fasern 2 angeordnet sind. In diesem Zustand ist das Implantat 1 vorzugsweise in einen Patienten einsetzbar.

Die Formgestaltung ist hierbei flexibel ausführbar. So verdeutlichen die Figuren 1 und 2 lediglich die Materialzusammensetzung, während das übergeordnete Implantat 1 derart auszugestalten ist, dass es das zu stützende Hartgewebe komplementär ergänzt.

Die Bioresorption des Implantats 1 ist dadurch erhöht, dass in dem Zustand, der in Fig. 2 dargestellt ist keine Phasengrenzen 4 mehr auftreten.

## Patentansprüche

1. Bioresorbierbares Implantat (1) zum Ergänzen oder Ersetzen von Hartgewebe und / oder Weichgewebe, mit zumindest einer verstärkenden Faser (2), die aus einer ersten Materialkomponente aufgebaut ist und die nach einer Vermischung mit einer zweiten Materialkomponente in einer Matrix (3) eingebettet ist, wobei das Material der ersten Materialkomponente zumindest eines der Elemente der Gruppe aus Seide, Chitosan, Collagen, Polycaprolacton, Poly-DL-Lactide, Polylactid-co-Glycolid, Polyclycolide, Polyurethane und Polypropylen enthält, **dadurch gekennzeichnet, dass** die zweite Materialkomponente zum Zeitpunkt der Vermischung mit der zumindest einen verstärkenden Faser (2) granulatartig oder pulverförmig vorliegt, wobei die Gesamtheit an Fasern (2) zumindest zwei der Elemente der Gruppe aus Seide, Chitosan, Collagen, Polycaprolacton, Poly-DL-Lactide, Polylactid-co-Glycolid, Polyclycolide, Polyurethane und Polypropylen enthält.

2. Bioresorbierbares Implantat (1) nach Anspruch 1, **dadurch gekennzeichnet, dass** die Matrix (3) aus der zweiten Materialkomponente und zumindest einer weiteren Materialkomponente aufgebaut ist.

3. Bioresorbierbares Implantat (1) nach einem der Ansprüche 1 oder 2, **dadurch gekennzeichnet, dass** die zweite Materialkomponente keramische phosphatbasierte Komponenten umfasst.

4. Bioresorbierbares Implantat (1) nach einem der Ansprüche 1 bis 3, **dadurch gekennzeichnet, dass** die Faser (2) zumindest eine Erhöhung und/oder Vertiefung aufweist, um eine Interaktionsoberfläche zwischen der Matrix (3) und der Faser (2) zu erhöhen.

5. Bioresorbierbares Implantat (1) nach einem der Ansprüche 1 bis 4, **dadurch gekennzeichnet, dass** in der Matrix (3) mehrere Festkörperpartikel angeordnet sind, die eine Steuerung einer Resorptionszeit ermöglichen, wobei die Festkörperpartikel einen Masseanteil von 5% bis 25% gemessen an der Masse des bioresorbierbaren Implantats (1) aufweisen.

6. Bioresorbierbares Implantat (1) nach einem der Ansprüche 1 bis 5, **dadurch gekennzeichnet, dass** eine Vermengung der einzelnen Materialkomponenten über eine generative, subtraktive und 3D-formgebende Fertigung ermöglicht ist.

7. Bioresorbierbares Implantat (1) nach einem der Ansprüche 1 bis 6, **dadurch gekennzeichnet, dass** die zum Zeitpunkt der Vermischung granulatartigen oder pulverförmigen Partikel der zweiten Materialkomponente nach einem vorbestimmten Zeitfenster eine ganzheitliche und ineinander übergehende Form annehmen.

8. Bioresorbierbares Implantat (1) nach einem der Ansprüche 1 bis 7, **dadurch gekennzeichnet, dass** die zum Zeitpunkt der Vermischung granulatartigen oder pulverförmigen Partikel der zweiten Materialkomponente zumindest eines der Elemente der Gruppe aus Magnesium, Calcium, Hydroxylapatit, alpha- und/oder beta-Tricalciumphosphat und Kalk enthält, um das Abbauverhaltung des bioresorbierbaren Implantats (1) gezielt zu beeinflussen.

9. Verfahren zum Herstellen eines bioresorbierbaren Implantats (1) nach einem der vorstehenden Ansprüche, mit den Schritten:
- Bereitstellen der zumindest einen Faser (2) aus der ersten Materialkomponente;
- Bereitstellen von granulatartigen oder pulverförmigen Partikeln der zweiten Material komponente;
- Vermischung der ersten Materialkomponente mit der zweiten Materialkomponente, um das bioresorbierbare Implantat (1) zu erhalten;
- Nachbearbeitung des bioresorbierbaren Implantats (1) in seiner Form und/oder Oberflächenbeschaffenheit, um komplementär zu dem zu ersetzenden Hartgewebe und / oder Weichgewebe ausgestaltet zu sein.

## Claims

1. A bioresorbable implant (1) for supplementing or replacing hard tissue and/or soft tissue, comprising at least one reinforcing fiber (2) which is made from a first material component and which, after being mixed with a second material component, is embedded in a matrix (3), wherein the material of the first material component contains at least one of the elements of the group consisting of silk, chitosan, collagen, polycaprolactone, poly(D,L-lactide), poly(lactide-co-glycolide), polyglycolide, polyurethane and polypropylene, **characterized in that** the second material component is present in granular or powdery form at the point in time at which the material component is mixed with the at least one reinforcing fiber (2), wherein the entirety of fibers contains at least two of the elements of the group consisting of silk, chitosan, collagen, polycaprolactone, poly(D,L-lactide), poly(lactide-co-glycolide), polyglycolide, polyurethane and polypropylene.

2. The bioresorbable implant (1) according to claim 1, **characterized in that** the matrix (3) is made from the second material component and at least one further material component.

3. The bioresorbable implant (1) according to one of claims 1 to 2, **characterized in that** the second material component comprises ceramic phosphate-based components.

4. The bioresorbable implant (1) according to one of claims 1 to 3, **characterized in that** the fiber (2) includes at least one elevation and/or recess to increase an interacting surface between the matrix (3) and the fiber (2).

5. The bioresorbable implant (1) according to one of claims 1 to 4, **characterized in that** several solid particles which enable a resorption time to be controlled are arranged in the matrix (3), wherein the solid particles include a mass percentage from 5% to 25% as measured by the mass of the bioresorbable implant (1).

6. The bioresorbable implant (1) according to one of claims 1 to 5, **characterized in that** mixing of the individual material components is enabled by generative, subtractive and 3D-shaping fabrication.

7. The bioresorbable implant (1) according to one of claims 1 to 6, **characterized in that** the particles of the second material component which are granular or powdery at the time of mixing adopt an integral and merging shape after a predetermined time window.

8. The bioresorbable implant (1) according to one of claims 1 to 7, **characterized in that** the particles of the second material component which are granular or powdery at the time of mixing contain at least one of the elements of the group consisting of magnesium, calcium, hydroxyapatite, alpha- and/or beta-tricalcium phosphate and lime to specifically influence the degradation behavior of the bioresorbable implant (1).

9. A method for producing a bioresorbable implant (1) according to one of the preceding claims, comprising the steps of:
- providing the at least one fiber (2) from the first material component;
- providing granular or powdery particles of the second material component;
- mixing the first material component with the second material component to obtain the bioresorbable implant (1);
- post processing the bioresorbable implant (1) as to its shape and/or surface texture so that it is configured to be complementary to the hard tissue and/or soft tissue subject to replacement.

## Revendications

1. Implant biorésorbable (1) pour compléter ou remplacer du tissu dur et/ou du tissu mou, avec au moins une fibre de renforcement (2) qui est constituée d'un premier composant de matériau et qui est intégrée dans une matrice (3) après un mélange avec un second composant de matériau, dans lequel le matériau du premier composant de matériau contient au moins un des éléments du groupe de la soie, du chitosane, du collagène, d'une polycaprolactone, d'un poly-DL-lactide, d'un polylactide-co-glycolide, d'un polyglycolide, d'un polyuréthane et d'un polypropylène, **caractérisé en ce que** le second composant de matériau se présente au moment du mélange avec l'au moins une fibre de renforcement (2) sous forme de granulat ou de poudre, dans lequel l'intégralité des fibres (2) contient au moins deux des éléments du groupe de la soie, du chitosane, du collagène, d'une polycaprolactone, d'un poly-DL-lactide, d'un polylactide-co-glycolide, d'un polyglycolide, d'un polyuréthane et d'un polypropylène.

2. Implant biorésorbable (1) selon la revendication 1, **caractérisé en ce que** la matrice (3) est constituée du second composant de matériau et d'au moins un autre composant de matériau.

3. Implant biorésorbable (1) selon l'une quelconque des revendications 1 ou 2, **caractérisé en ce que** le second composant de matériau comprend des composants céramiques à base de phosphate.

4. Implant biorésorbable (1) selon l'une quelconque des revendications 1 à 3, **caractérisé en ce que** les fibres (2) présente au moins une élévation et/ou une cavité afin d'augmenter une surface d'interaction entre la matrice (3) et la fibre (2).

5. Implant biorésorbable (1) selon l'une quelconque des revendications 1 à 4, **caractérisé en ce que** plusieurs particules de corps solides sont agencées dans la matrice (3), lesquelles permettent une commande d'un temps de résorption, dans lequel les particules de corps solides présentent une fraction massique de 5 % à 25 % mesurée au niveau de la masse de l'implant biorésorbable (1).

6. Implant biorésorbable (1) selon l'une quelconque des revendications 1 à 5, **caractérisé en ce qu'**un mélange des composants de matériau individuels est permis par le biais d'une fabrication générative, soustractive et par conformation en 3D.

7. Implant biorésorbable (1) selon l'une quelconque des revendications 1 à 6, **caractérisé en ce que** les particules en forme de granulat ou de poudre au moment du mélange du second composant de matériau après une fenêtre de temps prédéterminée adoptent une forme globale et passant l'une dans l'autre.

8. Implant biorésorbable (1) selon l'une quelconque des revendications 1 à 7, **caractérisé en ce que** les particules en forme de granulat ou de poudre au moment du mélange du second composant de matériau contiennent au moins un des éléments du groupe du magnésium, du calcium, de l'hydroxyapatite, du phosphate tricalcique alpha et/ou béta et de la chaux afin d'influencer de manière ciblée le comportement à la décomposition de l'implant biorésorbable (1).

9. Procédé de fabrication d'un implant biorésorbable (1) selon l'une quelconque des revendications précédentes, avec les étapes consistant à :
- fournir au moins une fibre (2) en un premier composant de matériau ;
- fournir des particules en forme de granulat ou de poudre du second composant de matériau ;
- mélanger le premier composant de matériau avec le second composant de matériau afin d'obtenir l'implant biorésorbable (1) ;
- traiter ultérieurement l'implant biorésorbable (1) dans sa forme et/ou état de surface afin d'être configuré de manière complémentaire au tissu dur et/ou tissu mou à remplacer.
